Europäisches Patentamt

European Patent Office

Office européen des brevets

(19)

(11) Veröffentlichungsnummer: **0 179 971**
A2

# (12) EUROPÄISCHE PATENTANMELDUNG

(21) Anmeldenummer: 85102032.1

(22) Anmeldetag: 23.02.85

(51) Int. Cl.⁴: **A 61 B 7/02**

(30) Priorität: 31.10.84 DE 8431892 U

(43) Veröffentlichungstag der Anmeldung: 07.05.86
Patentblatt 86/19

(84) Benannte Vertragsstaaten: **AT BE CH DE FR GB IT LI NL SE**

(71) Anmelder: **Nowak, Peter, Kiebitzmoor 3, D-2153 Neu Wulmstorf (DE)**
Anmelder: **Grabe, Karl-Georg, Wilseder Ring 97 F, D-2100 Hamburg 90 (DE)**

(72) Erfinder: **Nowak, Peter, Kiebitzmoor 3, D-2153 Neu Wulmstorf (DE)**
Erfinder: **Grabe, Karl-Georg, Wilseder Ring 97 F, D-2100 Hamburg 90 (DE)**

(74) Vertreter: **Schmidt-Bogatzky, Jürgen, Dr. Ing., Schlossmühlendamm 1, D-2100 Hamburg 90 (DE)**

(54) Vorrichtung zur Temperierung von Stethoskopen.

(57) Die Erfindung betrifft eine Vorrichtung zur Temperierung von Stethoskopen. Sie besteht aus einem Gehäuse (2) in dem eine Heizeinrichtung (3) angeordnet ist. Auf einer Gehäuseaußenfläche befindet sich ein Halteglied (4) für ein Stethoskop. In dem Gehäuse (2) ist ein elektronischer Temperaturregler (6) vorgesehen. Die Heizeinrichtung (3) steht über ein Anschlußstück (7) mit einem Anschlußkabel (9) in Verbindung, das durch eine Gehäusewand (8) geführt ist.

0179971

Die Erfindung betrifft eine Vorrichtung zur Temperierung von Stethoskopen.

Bei der ärztlichen Untersuchung von Kindern und älteren Patienten besteht das Problem, daß das Auflegen von kalten Stethoskopen von den Patienten als äußerst unangenehm empfunden wird und häufig zu Schreckreaktionen führt.

Die Aufgabe der Erfindung besteht darin, eine einfach zu bedienende Vorrichtung zu schaffen, mittels derer Stethoskope entsprechend der Körpertemperatur temperiert werden können.

Erfindungsgemäß erfolgt die Lösung der Aufgabe durch eine in einem Gehäuse angeordnete Heizeinrichtung und mindestens einem auf einer Gehäuseaußenfläche angeordneten Halteglied für ein Stethoskop. Durch ein hierdurch auf Körpertemperatur angewärmtes Stethoskop verlieren insbesondere Kinder wie auch ältere Patienten ihre Angst vor dem Gerät.

Weitere Merkmale der Erfindung werden in den Unteransprüchen beschrieben.

In den Zeichnungen ist ein Ausführungsbeispiel der Erfindung dargestellt, das nachstehend erläutert wird. Es zeigt

Fig. 1          die erfindungsgemäße Vorrichtung in einer Vorderansicht

Fig. 2          die Vorrichtung nach Fig. 1 in einer Draufsicht

Fig. 3          die Vorrichtung nach Fig. 1 in einer Seitenansicht im Schnitt

Fig. 4    die Vorrichtung nach Fig. 3 in einer Draufsicht im Ausschnitt im Schnitt A-A.

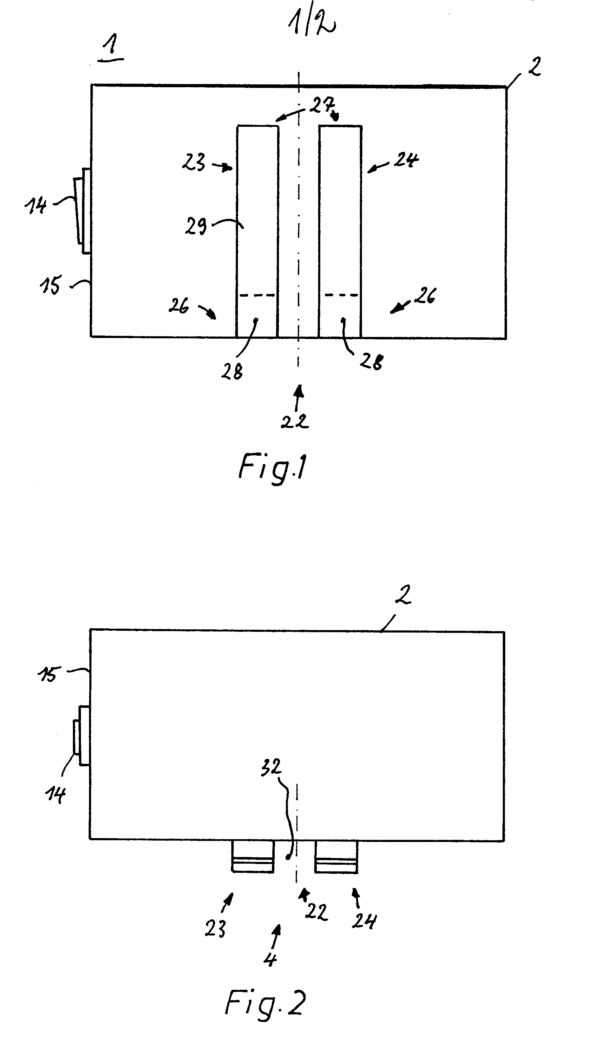

Die Vorrichtung 1 zur Temperierung von Stethoskopen 5 besteht aus einem Gehäuse 2, an dessen einer Gehäusewand 15 ein Ein-/Ausschalter 14 für eine in dem Gehäuse 2 befindliche Heizeinrichtung 3 und an dessen einer anderen Gehäusewand 18 ein Halteglied 4 für das Stethoskop 5 angeordnet ist (Fig. 1 bis 3).

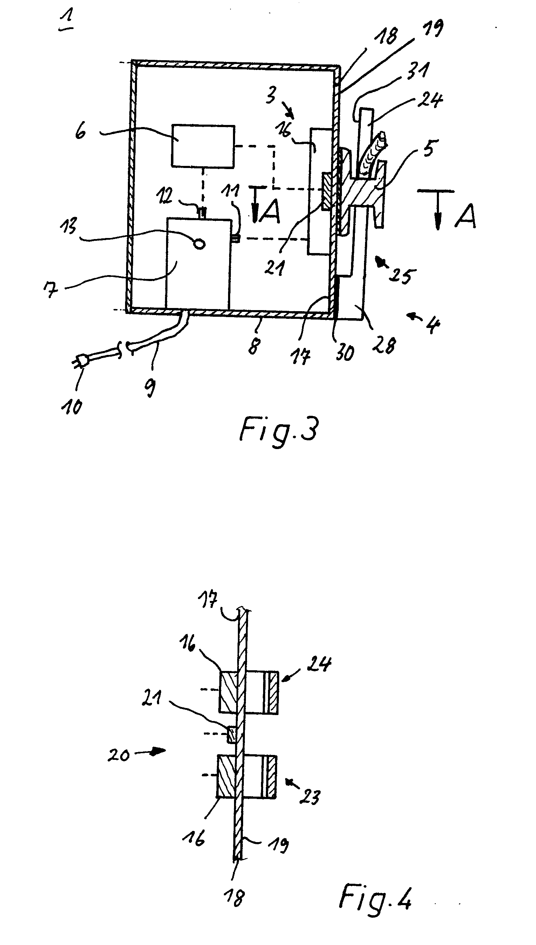

In dem Gehäuse ist ein Anschlußstück 7 für ein Anschlußkabel 9 ausgebildet, das durch die Gehäusewand 8 geführt ist und an dem freien Endabschnitt einen Stecker 10 aufweist. Mittels dieses Steckers 10 kann die Vorrichtung 1 an eine Energieversorgungquelle angeschlossen werden. Das Anschlußstück 7 weist Verbindungsglieder 11, 12, 13 auf, mittels derer über in Fig. 3 durch Strichlinien angedeutete Verbindungskabel die Heizeinrichtung 3, der Temperaturregler 6 und der Ein-/Ausschalter 13 mit dem Anschlußstück 7 verbunden ist. An der Innenfläche 17 der das Halteglied 4 tragenden Gehäusewand 18 sind als Heizeinrichtung 3 zwei Heizwiderstände 16 angeordnet. Zwischen diesen Heizwiderständen 16, die die Heizfläche 20 bilden, befindet sich ein Temperatursensor 21 des Temperaturreglers 6 (Fig. 4).

Das Halteglied 4 ist als nach oben offene Haltegabel 22 ausgebildet. Die Haltegabel 22 besteht aus zwei Gabelstücken 23, 24, die als streifenförmige Zuschnitte 25 ausgebildet sind. Jeder Zuschnitt 25 weist an dem unteren Endabschnitt 26 einen flächigen Absatz 28 auf, an den sich ein flächiger elastischer Gabelzinken 29 anschließt. Die Gabelstücke 23, 24 können aus Kunststoff oder Metall bestehen. Die Absätze 28 sind mittels einer Klebverbindung 30 mit der Gehäusewand 18 verbunden. Es ist auch möglich, statt der Klebverbindung 30 andere geeignete Verbindungsmittel vorzusehen. Die Gabelzinken 29 sind im Querschnitt so ausgebildet, daß sich ihre der Gehäusewand 18 zugewandten Flächen 31 in einem kleinen Winkel zur Gabelspitze 27 von der Gehäusewand 18 erweiternd sind. Hierdurch kann über den Bereich der Gabelspitzen 27 in den zwischen den Gabelstücken 23, 24 ausgebildeten Raum 32 das Stethoskop 5 leicht eingeführt werden, das dann im unteren Bereich der Gabelstücke 23, 24 zwischen den Flächen 31 der Gabelzinken 29 und der Gehäusewand 18 festklemmt. Es ist auch möglich, an einem Gehäuse 2 statt eines Halteglieds 4 mehrere Halteglieder 4 vorzusehen.

Der Temperaturregler 6 wird vorzugsweise als elektronischer Zweipunktregler ausgebildet, der es ermöglicht, die Außenfläche 19 der Gehäusewand 18 im Bereich des Halteglieds 4 mittels der Heizwiderstände 16 auf eine Temperatur von 37°C bis 38°C aufzuheizen. Der Temperaturregler 6 kann mit einem Trimmer versehen werden, durch den sich der Temperaturbereich variieren läßt. Durch diesen elektronischen Temperaturregler 6 ist es möglich, den dem Stethoskop 5 zugewandten Flächenabschnitt der Außenfläche 19 ständig auf einer Betriebstemperatur von ca. 37,5°C zu halten, sodaß sich ein in das Halteglied 4 eingeführtes Stethoskop in weniger als 10 Sekunden anwärmen läßt.

**P A T E N T A N S P R Ü C H E**

1. Vorrichtung zur Temperierung von Stethoskopen, gekennzeichnet durch eine in einem Gehäuse (2) angeordnete Heizeinrichtung (3) und einem auf einer Gehäuseaußenfläche angeordneten Halteglied (4) für ein Stethoskop (5).

2. Vorrichtung nach Anspruch 1, dadurch gekennzeichnet, daß in dem Gehäuse (2) ein elektronischer Temperaturregler (6) angeordnet ist.

3. Vorrichtung nach Anspruch 1 und 2, dadurch gekennzeichnet, daß in dem Gehäuse (2) ein Anschlußstück (7) für ein durch eine Gehäusewandung (8) geführtes Anschlußkabel (9) mit Verbindungsgliedern (11, 12, 13) für die Heizeinrichtung (3), den Temperaturregler und einen in einer weiteren Gehäusewand (15) angeordneten Ein-/Ausschalter (14) ausgebildet ist.

4. Vorrichtung nach Anspruch 1 bis 3, dadurch gekennzeichnet, daß die Heizeinrichtung (3) aus Heizwiderständen (16) besteht, die an der Innenfläche (17) der Gehäusewand (18) angeordnet sind, an deren Außenfläche (19) sich das Halteglied (4) befindet.

5. Vorrichtung nach Anspruch 4, dadurch gekennzeichnet, daß an der Innenfläche (17) der Gehäusewand (18) im Bereich der durch die Heizwiderstände (16) gebildeten Heizfläche (20) ein Temperatursensor (21) des Temperaturreglers (6) angeordnet ist.

6. Vorrichtung nach Anspruch 1 bis 5, dadurch gekennzeichnet, daß das Halteglied (4) als nach oben offene Haltegabel (22) ausgebildet ist.

7. Vorrichtung nach Anspruch 6, dadurch gekennzeichnet, daß die Haltegabel (22) aus zwei Gabelstücken (23, 24) besteht, die als streifenförmige Zuschnitte (25) mit einem flächigen Absatz (28) an dem unteren Endabschnitt (26) ausgebildet sind.

8. Vorrichtung nach Anspruch 6 und 7, dadurch gekennzeichnet, daß die an die Absätze (28) anschließenden flächigen Gabelzinken (29) elastisch ausgebildet sind.

9. Vorrichtung nach Anspruch 6 und 7 dadurch gekennzeichnet, daß die Gabelstücke (23, 24) aus Kunststoff oder Metall bestehen.

10. Vorrichtung nach Anspruch 7, dadurch gekennzeichnet, daß die Absätze (28) mittels einer Klebverbindung (30) mit der Gehäusewand (18) verbunden sind.

11. Vorrichtung nach Anspruch 6 bis 10, dadurch gekennzeichnet, daß die der Gehäusewand (18) zugewandte Fläche (31) der Gabelzinken (29) in einem kleinen Winkel sich zur Gabelspitze (27) von der Gehäusewand (18) erweiternd ausgebildet sind.

0179971

Fig.1

Fig.2

0179971

Fig.3

Fig.4